# EUROPÄISCHE PATENTSCHRIFT

(11) **EP 2 405 955 B1**
(45) Veröffentlichungstag und Bekanntmachung des Hinweises auf die Patenterteilung: **16.11.2016**
(21) Anmeldenummer: 10708928.6
(22) Anmeldetag: 09.03.2010
(51) Int. Cl.: A61M 1/36, A61M 1/14

(54) **BLUTBEHANDLUNGVORRICHTUNG MIT VORRICHTUNG VERBINDEN EINER EXTERNEN FUNKTIONSEINRICHTUNG MIT EINER ANORDNUNG, ANORDNUNG AUFWEISEND EINE SOLCHE VORRICHTUNG UND VERFAHREN ZUM VERBINDEN**
BLOOD TREATMENT DEVICE WITH DEVICE FOR CONNECTING AN EXTERNAL FUNCTION DEVICE WITH A DEVICE, SYSTEM COMPRISING SUCH A DEVICE AND METHOD FOR THE CONNECTION
DISPOSITIF DE TRAITEMENT DU SANG AVEC DISPOSITIF DE RACCORDEMENT D'UN DISPOSITIF DE FONCTION EXTERNE AVEC UN DISPOSITIF, SYSTÈME COMPRENANT UN TEL DISPOSITIF ET PROCÉDÉ POUR LA CONNEXION

(30) Priorität: 10.03.2009 DE 102009012633
(43) Veröffentlichungstag der Anmeldung: 18.01.2012
(73) Patentinhaber: Fresenius Medical Care Deutschland GmbH, 61352 Bad Homburg (DE)
(72) Erfinder: HÄCKER, Jürgen, 61267 Neu-Anspach (DE); LAPP, Uwe, 35510 Butzbach (DE); WÄBER, Udo, 63071 Offenbach (DE)
(74) Vertreter: Bobbert & Partner Patentanwälte PartmbB
(86) Internationale Anmeldenummer: PCT/EP2010/001464
(87) Internationale Veröffentlichungsnummer: WO 2010/102790

(56) Entgegenhaltungen:
- WO-A1-01/17653
- US-A1- 2007 276 328
- US-A1- 2009 012 448

## Beschreibung

Die vorliegende Erfindung betrifft eine Vorrichtung zum Verbinden wenigstens einer externen Funktionseinrichtung mit einer Anordnung gemäß dem Oberbegriff des Anspruchs 1. Sie betrifft ferner ein Verfahren zum Verbinden wenigstens einer externen Funktionseinrichtung mit einer Anordnung unter Verwenden der erfindungsgemäßen Vorrichtung gemäß dem Oberbegriff des Anspruchs 13, und eine Anordnung gemäß dem Anspruch 14, die eine erfindungsgemäße Vorrichtung aufweist.

Bei technischen Anordnungen, wie beispielsweise Behandlungsmaschinen der Medizintechnik, labortechnischen Anordnungen oder auch Anordnungen zur Nahrungsmittelherstellung, ist oftmals eine funktionelle Ankopplung externer Funktionseinrichtungen an die Anordnung vorgesehen. Ein Beispiel einer solchen externen Funktionseinrichtung ist eine Disposable-Kassette, wie sie in der DE 10 2007 042 964 beschrieben ist.

Eine funktionelle Ankopplung derartiger externer Funktionseinrichtungen setzt üblicherweise deren korrekte Positionierung und/oder Fixierung auf bzw. an der Anordnung voraus, damit anordnungsseitige Sensoren und Aktoren mit der externen Funktionseinrichtung fehlerfrei zusammenwirken können.

Aufgabe der vorliegenden Erfindung ist es, eine weitere Vorrichtung zum Verbinden wenigstens einer externen Funktionseinrichtung mit einer Anordnung bereitzustellen. Ferner soll ein Verfahren zum Verbinden wenigstens einer externen Funktionseinrichtung sowie eine Anordnung, welche eine derartige Vorrichtung aufweist, angegeben werden.

Die erfindungsgemäße Aufgabe wird durch eine Vorrichtung mit den Merkmalen des Anspruchs 1 gelöst.

Die erfindungsgemäße Vorrichtung dient zum Verbinden oder zur Verbindung, insbesondere zum Ankoppeln, wenigstens einer externen Funktionseinrichtung mit einer Anordnung und weist dazu wenigstens eine Aufnahmeeinrichtung mit wenigstens einem ersten Kontaktabschnitt und wenigstens einem zweiten Kontaktabschnitt auf, zwischen welchen die externe Funktionseinrichtung aufgenommen werden kann. Dabei ist wenigstens der erste Kontaktabschnitt und/oder der zweite Kontaktabschnitt bewegbar angeordnet, vorzugsweise gelagert. Unter "bewegbar" ist vorzugsweise eine selbstnachführende Bewegbarkeit oder Beweglichkeit des bewegbaren Kontaktabschnitts zu verstehen.

Im Sinne der vorliegenden Erfindung kann vom Begriff "bewegbar" jedoch eine Beweglichkeit, wie beispielsweise das bloße Schwenken um eine Aufhängung an einem Scharnier, wie beispielsweise das bekannte Schwenken einer Tür, ausgeschlossen sein. Diese Bedeutung kann von der vorliegenden Erfindung im Zusammenhang mit dem Begriff "bewegbar" alternativ jedoch explizit umfasst sein.

Der erste und/oder der zweite Kontaktabschnitt können derart vorgesehen sein, dass sie pendelbar angeordnet, vorzugsweise gelagert, sind, also zumindest beim Vorgang des Verbindens in wenigstens einer Richtung pendeln können.

Eine "externe Funktionseinrichtung", wie sie hierin verwendet wird, kann ein Wärmetauscher, eine Messeinrichtung, eine multifunktionale Disposable-Kassette oder dergleichen sein.

Eine erfindungsgemäß in Betracht gezogene externe Funktionseinrichtung kann zum Leiten medizinischer Fluide, wie Blut, Substituatflüssigkeit oder dergleichen geeignet sein. Eine derartige externe Funktionseinrichtung kann beispielsweise eine blutführende Kassette sein. Sie kann Teile eines extrakorporalen Blutkreislaufs enthalten. Insbesondere kann sie als eine Disposable-Kassette ausgestaltet sein, wie sie in der DE 10 2007 042 964 beschrieben ist. Deren gesamte diesbezügliche Offenbarung wird durch Bezugnahme Teil der vorliegenden Unterlagen. Dabei gilt im Zusammenhang mit der vorliegenden Offenbarung, dass die Ausdrücke "kann aufweisen", "kann sein" und dergleichen synonym zu den hier und auch an anderer Stelle ebenfalls verwendeten Ausdrücken "weist bevorzugt auf", "ist bevorzugt" und dergleichen zu verstehen sind.

Die externe Funktionseinrichtung kann vorzugsweise Energie, Messwerte und/oder mechanische Bewegungen und Kräfte auf die Anordnung übertragen oder von dieser empfangen sowie in Fluidverbindung mit der Anordnung stehen. Sie kann von der Anordnung alternativ jedoch auch nur gehalten werden und mit dieser nicht in Wechselwirkung und/oder Signalkommunikation und/oder Fluidverbindung stehen.

Die aufzunehmende oder zu verbindende externe Funktionseinrichtung kann an wenigstens einer Oberfläche eine Abdeckung aufweisen. Eine solche Abdeckung kann eine Folie sein. Die Folie kann zum Aufnehmen von Sensoren und/oder Aktoren und/oder Leitungen und/oder Teilen derselben vorgesehen sein. Die Folie kann Öffnungen zum Aufnehmen derartiger Komponenten aufweisen und/oder derartige Komponenten können in die Folie und/oder einen Folienverbund integriert sein.

Eine "Anordnung" im Sinne der vorliegenden Erfindung ist eine medizintechnische Anordnung, wie eine Blutbehandlungsvorrichtung, zum Beispiel eine Dialysevorrichtung, eine Hämodialysevorrichtung, eine Hämodiafiltrationsvorrichtung oder dergleichen.

Der Begriff "Verbinden", wie er hierin verwendet wird, kann ein funktionelles und/oder mechanisches Verbinden der externen Funktionseinrichtung mit der Anordnung bezeichnen oder umfassen.

Der Begriff "Ankoppeln", wie er hierin verwendet wird, kann ein funktionelles und/oder mechanisches Verbinden der externen Funktionseinrichtung mit einem Koppelpartner auf der Seite der Anordnung mit einer Wechselwirkung oder Signalkommunikation oder Fluidverbindung zwischen externer Funktionseinrichtung und Anordnung im angekoppelten Zustand der externen Funktionseinrichtung bezeichnen oder umfassen. Ein "Koppelpartner der Anordnung" kann zum Beispiel eine Messeinrichtung, wie beispielsweise ein Sensor, sein. Er kann als eine Fluidleitung und dergleichen ausgestaltet sein.

Eine "Aufnahmeeinrichtung" im Sinne der vorliegenden Erfindung ist eine Einrichtung, die zum Aufnehmen wenigstens einer externen Funktionseinrichtung geeignet ist. Dabei kann Aufnehmen ein Halten, ein Umfassen, ein Umgreifen, ein Bedecken der externen Funktionseinrichtung und dergleichen sowie Kombinationen hieraus bedeuten.

Der "erste Kontaktabschnitt" der Aufnahmeeinrichtung ist bevorzugt an der erfindungsgemäßen Vorrichtung selbst oder an einer Anordnung vorgesehen. Er kann beweglich, z.B. in vertikaler und/oder horizontaler oder anderer Richtung - jeweils bezogen auf den Erdmittelpunkt - pendelnd, innerhalb der Vorrichtung oder der Anordnung angeordnet sein.

Der erste Kontaktabschnitt kann verschiedene Funktionen übernehmen. So kann er beispielsweise zum Ausrichten der externen Funktionseinrichtung, zu ihrer Verrastung und/oder zur Übertragung einer Verpresskraft auf die externe Funktionseinrichtung sowie Kombinationen der vorgenannten Funktionen dienen.

Der erste Kontaktabschnitt kann weitere Einrichtungen zum Unterstützen eines Verpressens der externen Funktionseinrichtung und/oder Halten derselben, wie beispielsweise eine Verpresseinrichtung und/oder eine Verriegelungseinrichtung, aufweisen oder mit solchen Einrichtungen verbunden sein.

Der erste Kontaktabschnitt kann eine "Ankoppelfläche", eine "Aktivator-/Sensor-Platte" (kurz "AS-Platte") oder ein Maschineninterface sein.

Der Begriff "Ankoppelfläche" kann beispielsweise wenigstens einen Abschnitt einer Oberseite eines Trägerelements und/oder eines Auflage- oder Stützelements und/oder einer Messeinrichtung oder dergleichen der Anordnung und/oder der Vorrichtung bezeichnen.

Eine "AS-Platte" kann Aktoren und/oder Sensoren und/oder Leitungen zum Ankoppeln an die externe Funktionseinrichtung oder Teile derselben aufweisen.

Mittels der Ankoppelfläche, welche flach bzw. eben sein kann, aber nicht sein muss, kann eine Wechselwirkung oder Signalkommunikation zwischen der externen Funktionseinrichtung und einem Abschnitt der Vorrichtung oder der Anordnung hergestellt werden.

"Aktoren" im hier gebrauchten Sinn schließen, ohne darauf beschränkt zu sein, mechanische und/oder pneumatische und/oder elektrische Komponenten, wie beispielsweise Ventile, Stell- und/oder Regelglieder, Stellmotoren, Druckkolben, und dergleichen ein.

"Sensoren", wie sie hier bezeichnet sind, schließen, wiederum ohne darauf beschränkt zu sein, elektrische, optische, akustische, virtuelle und/oder digitale Sensoren, Messwertaufnehmer und/oder Messwertfühler und dergleichen ein.

Ein Sensor kann bestimmte physikalische und/oder chemische Eigenschaften und/oder eine Änderung derselben, wie eine Zunahme oder eine Abnahme eines Effektes, einer Ausprägung, einer Beschaffenheit und dergleichen, wie beispielsweise Temperatur, Druck, Feuchtigkeit, optische Signale, wie beispielsweise Helligkeit und/oder eine optische Änderung einer Zusammensetzung, Wärmestrahlung, Schall, Durchflussmengen, und/oder die stoffliche Beschaffenheit seiner Umgebung qualitativ oder quantitativ erfassen.

Derartige Sensoren können als passive Sensoren oder aktive Sensoren ausgelegt sein.

Leitungen, wie sie erfindungsgemäß eingesetzt werden können, können, ohne darauf beschränkt zu sein, Fluidleitungen, wie beispielsweise blutführende Kanäle, Substituatleitungen, elektrische Leitungen, Leitungen zur Signalkommunikation, wie beispielsweise Glasfaserkabel, und dergleichen umfassen.

Der "zweite Kontaktabschnitt" der Aufnahmeeinrichtung kann wie der erste Kontaktabschnitt ausgestaltet sein und vorzugsweise dieselben Funktionen übernehmen.

Der "zweite Kontaktabschnitt" kann eine Andruckplatte sein. Er kann flach, stufig, wellig oder auf andere Weise ausgestaltet sein. Er kann punktförmig oder flächig mit der externen Funktionseinrichtung in Kontakt treten. Er kann einen Abschnitt aufweisen, welcher in seiner Geometrie einer Erstreckung der anzukoppelnden externen Funktionseinrichtung - im angekoppelten oder im nicht angekoppelten Zustand - entspricht. Dies kann deren Aufnahme und/oder Ausrichtung erleichtern oder begünstigen.

Der zweite Kontaktabschnitt kann beweglich oder starr ausgestaltet sein.

Der zweite Kontaktabschnitt kann vorgesehen sein, um mittelbar oder unmittelbar mit der externen Funktionseinrichtung in Kontakt zu treten.

Der zweite Kontaktabschnitt kann eine Tür oder ein Deckel zum Verschließen eines Inneren der erfindungsgemäßen Vorrichtung oder der Anordnung oder ein Abschnitt jeweils hiervon sein.

Vorteilhafte Weiterentwicklungen der vorliegenden Erfindung sind jeweils Gegenstand der Unteransprüche.

In einer bevorzugten erfindungsgemäßen Ausführungsform weist die Vorrichtung wenigstens eine umlaufend ausgestaltete Trägereinrichtung auf. Der erste Kontaktabschnitt kann in einem Inneren der Trägereinrichtung gelagert sein.

Eine solche "Trägereinrichtung" kann eine stabile Trägereinrichtung, wie beispielsweise ein Trägerrahmen, sein. Er kann Teil der Aufnahmeeinrichtung zum Aufnehmen der wenigstens einen externen Funktionseinrichtung sein.

Die Trägereinrichtung kann kraft- und/oder form- und/oder stoffschlüssig mit der Anordnung verbunden sein.

Die Trägereinrichtung kann mit dem ersten und/oder dem zweiten Kontaktabschnitt verbunden sein.

Insbesondere kann der erste Kontaktabschnitt im Fall seiner Ausgestaltung als AS-Platte in Richtung zum zweiten Kontaktabschnitt und/oder der externen Funktionseinrichtung hin an einer Oberfläche eines von der Trägereinrichtung umschlossenen Abschnitts bzw. eines Inneren angeordnet sein.

Der erste Kontaktabschnitt kann derart im Inneren der Trägereinrichtung angeordnet sein, dass eine Oberfläche des ersten Kontaktabschnitts und eine Oberfläche der Trägereinrichtung in einer Ebene liegen. Insbesondere können die in Richtung des zweiten Kontaktabschnitts und/oder der externen Funktionseinrichtung gerichtete Oberfläche des ersten Kontaktabschnitts und die in Richtung des zweiten Kontaktabschnitts und/oder der externen Funktionseinrichtung gerichtete Oberfläche der Trägereinrichtung in einer Ebene liegen.

Zwischen dem im Inneren der Trägereinrichtung angeordneten ersten Kontaktabschnitt und der Trägereinrichtung kann eine Öffnung bzw. ein freier Raum vorhanden sein. Eine solche Öffnung kann einen - insbesondere umlaufenden - Spalt bilden.

Der erste Kontaktabschnitt kann im Inneren der Trägereinrichtung beweglich gelagert sein. Der erste Kontaktabschnitt kann pendelnd, im Besonderen entlang einer auf seiner Oberfläche senkrecht stehenden Achse, pendelnd angeordnet sein.

In einer weiter bevorzugten Ausführungsform ist wenigstens einer der beiden Kontaktabschnitte um wenigstens eine Drehachse drehbar gelagert. Die dabei erzielbare Drehbarkeit oder die Pendelmöglichkeit sind als Formen einer Bewegbarkeit im Sinne der vorliegenden Erfindung zu verstehen.

Wenn im Folgenden von einer Drehachse oder Achse die Rede ist, kann hierunter erfindungsgemäß auch eine Mehrzahl von Achsen zu verstehen sein, wann immer der Fachmann dies als technisch möglich erkennt. Auch Dreh- oder Pendelbewegungen, welche zugleich in mehr als einer Achsenrichtung möglich sind, werden im Folgenden der besseren Lesbarkeit wegen als solche in einer Achsenrichtung subsumiert. Dies soll die vorliegende Erfindung jedoch nicht einschränken.

Dabei kann die Drehachse eine "körperliche" Drehachse sein. Eine körperliche Drehachse im Sinne der vorliegenden Erfindung kann eine gegenständliche Achse sein. Sie kann eine durch Verbinden wenigstens eines Kontaktabschnittes mit der Trägereinrichtung gebildete, räumlich starre Achse sein. Sie kann horizontal oder vertikal, bezogen auf eine während der Verwendung der Anordnung übliche Ausrichtung der Anordnung oder bezogen auf den Erdmittelpunkt, ausgerichtet sein.

Eine körperliche Achse kann als eine feststehende Achse, oder als eine sich drehende Welle ausgestaltet sein. Vorzugsweise weist sie wenigstens ein geeignetes Lager auf. Sie kann jeweils durch eine Lagerverbindung des ersten Kontaktabschnitts und/oder des zweiten Kontaktabschnitts mit der Trägereinrichtung und/oder einem anderen Bauteil der Anordnung ausgestaltet sein.

Beispiele für derartige Lagerverbindungen schließen, ohne darauf beschränkt zu sein, Lagerungsstifte, die in geeignete Lagerungsaufnahmeeinrichtungen eingebracht und/oder eingelegt werden, Kugellager, Rollenlager, Wälzlager, Gleitlager und dergleichen ein.

Unter "Achse" kann erfindungsgemäß alternativ auch eine Raumachse oder eine Achse in einer Raumrichtung verstanden werden. Sie ist in diesem Fall somit virtuell und im Gegensatz zur zuvor erläuterten gegenständlichen Achse nicht greifbar.

Die Achse im Sinne der vorliegenden Erfindung kann als ein Drehzentrum oder ein Drehmittelpunkt zu verstehen sein, um welche der Kontaktabschnitt gedreht, also bewegt werden kann. Die Bewegbarkeit des Kontaktabschnitts um die Achse kann eine Pendelbewegung der externen Funktionseinrichtung bei ihrem Verbinden mit der Anordnung oder der Vorrichtung ermöglichen.

Die Achse kann in jeder Ausführungsform durch den Kontaktabschnitt verlaufen. Sie kann jedoch auch außerhalb verlaufen.

Die Achse kann in einem Verbindungszustand der externen Funktionseinrichtung durch das Innere der Vorrichtung und/oder durch das Innere der Anordnung verlaufen.

Die Achse kann vorzugsweise derart verlaufen, dass sie eine Dreh- oder Pendelbewegung um sie herum allein durch ein Drehen oder Pendeln des betreffenden Kontaktabschnitts, nicht aber weiterer Bauelemente der Vorrichtung oder Anordnung, ermöglicht. Dies ist insbesondere dann vorzugsweise möglich, wenn der drehbare Kontaktabschnitt die Ankoppelfläche für die externe Funktionseinrichtung bzw. das sogenannte Maschineninterface ist.

Die Achse kann derart angeordnet sein, dass sie Ausgleichbewegungen wenigstens eines Kontaktabschnitts zum Ausgleichen von Toleranzen, Passungenauigkeiten, Verschleißerscheinungen usw. ermöglicht, darüber hinaus aber keine Drehbewegung eines Bauteils ermöglicht.

In einer bevorzugten Ausführungsform ist wenigstens einer der beiden Kontaktabschnitte mittels wenigstens eines Kugelgelenks gelagert.

Ein mittels Kugelgelenk gelagerter Kontaktabschnitt kann in alle Raumrichtungen beweglich sein. Beispielsweise kann der erste Kontaktabschnitt auf diese Weise in allen Raumrichtungen drehbar gelagert sein.

In einer weiter bevorzugten Ausführungsform weisen sowohl wenigstens der erste als auch der zweite Kontaktabschnitt Drehachsen auf. Vorzugsweise sind ihre Drehachsen (alle oder nur einige davon) parallel zueinander angeordnet. Die beiden Drehachsen können unabhängig voneinander als gegenständliche, körperliche, feste oder virtuelle Drehachse ausgebildet sein. Sie können gleich oder voneinander verschieden ausgestaltet sein.

In einer weiter bevorzugten Ausführungsform ist vorgesehen, dass wenigstens eine Kontaktfläche des ersten und/oder des zweiten Kontaktabschnitts und wenigstens eine Kontaktfläche der externen Funktionseinrichtung so angeordnet werden, dass sie in einem angekoppelten Zustand der externen Funktionseinrichtung plan oder im Wesentlichen plan aufeinander zum Liegen kommen.

Eine "Kontaktfläche" des ersten und/oder des zweiten Kontaktabschnitts und eine Kontaktfläche der externen Funktionseinrichtung bezeichnen erfindungsgemäß diejenigen Oberflächen des jeweiligen Kontaktabschnitts und der externen Funktionseinrichtung, die einander im angekoppelten Zustand der externen Funktionseinrichtung an der Anordnung jeweils gegenüberliegen.

Der Begriff "plan", wie er erfindungsgemäß zu verstehen ist, soll das ebene oder im Wesentlichen ebene Aneinanderliegen der Kontaktabschnitte mit der externen Funktionseinrichtung bezeichnen. Dabei können der erste und der zweite Kontaktabschnitt, im Verbindungszustand derart zueinander ausgerichtet sein können, dass ihre jeweils einander gegenüber liegenden Flächen zueinander parallel oder im Wesentlichen parallel sind.

Auf diese Weise kann es vorteilhaft möglich sein, ein mögliches Verkanten der Kontaktabschnitte und der externen Funktionseinrichtung während des Vorgangs des Ankoppelns zu vermeiden. Eine optimale funktionelle Ankopplung der Komponenten der externen Funktionseinrichtung kann somit vorteilhaft sichergestellt werden. Übermäßige Materialbeanspruchung kann vorteilhaft vermieden werden.

Ein "angekoppelter Zustand" der externen Funktionseinrichtung bezeichnet ebenso wie ein "Verbindungszustand" erfindungsgemäß einen Zustand, in dem die externe Funktionseinrichtung in ihrem Gebrauchszustand funktionell mit der Anordnung verbunden ist. Ein solcher Zustand kann zum Ausführen einer Funktion, wie beispielsweise einer Behandlung medizinischer Fluide, wie einer Blutbehandlung, vorgesehen sein.

In einer weiteren bevorzugten Ausführungsform weist die erfindungsgemäße Vorrichtung wenigstens eine Verpresseinrichtung zum Verpressen der externen Funktionseinrichtung zwischen dem ersten Kontaktabschnitt und dem zweiten Kontaktabschnitt auf.

Eine "Verpresseinrichtung" zur Verwendung in der vorliegenden Erfindung kann ein Verpressaktor, eine elektrische Verpresseinrichtung, ein Stellmotor, eine pneumatische Verpresseinrichtung, ein Ventil, ein Kolben und dergleichen sein oder aufweisen sowie Kombinationen hiervon.

Die Verpresseinrichtung kann mit der Trägereinrichtung und/oder dem ersten Kontaktabschnitt und/oder dem zweiten Kontaktabschnitt und/oder einem anderen Bauteil der Vorrichtung und/oder der Anordnung verbunden sein. Sie kann kraft- und/oder formschlüssig mit wenigstens einer der vorstehend genannten Komponenten verbunden sein.

Die Verpresseinrichtung kann eine Verpresskraft zum Verpressen der externen Funktionseinrichtung zwischen den Kontaktabschnitten aufbringen und/oder übertragen. Eine derartige "Verpresskraft kann eine pneumatische Kraft, eine elektromotorische Kraft, magnetische Kraft, induktive Kraft und dergleichen sein.

Die Verpresseinrichtung kann zum Aufrechterhalten des verpressten Zustands der externen Funktionseinrichtung zwischen den beiden Kontaktabschnitten über eine Zeitdauer hinweg ausgelegt sein. Sie kann entsprechende Einrichtungen zum Aufrechterhalten, Steuern oder Regeln der Verpresskraft aufweisen.

Die Verpresseinrichtung kann ausgestaltet sein, um die Dichtigkeit der externen Funktionseinrichtung durch korrekten Sitz derselben auf einem der Kontaktabschnitte - beispielsweise in Gestalt einer Ankoppelebene oder eines Maschineninterface - sicherzustellen.

In einer weiteren bevorzugten Ausführungsform kann die Vorrichtung ferner wenigstens eine Verriegelungseinrichtung zum Fixieren des verpressten Zustands der externen Funktionseinrichtung zwischen dem ersten Kontaktabschnitt und dem zweiten Kontaktabschnitt aufweisen.

Eine "Verriegelungseinrichtung" im Sinne der vorliegenden Erfindung, kann ein Riegel, ein Schloss, eine Sperre, eine Lasche, eine Steckverbindung, eine Verrasteinrichtung und dergleichen sein.

Die Verriegelungseinrichtung kann eine weitere zum Verpressen erforderliche Kraft aufbringen und eine Gesamtverpresskraft erhöhen. Hierzu kann sie eine entsprechende Einrichtung aufweisen. Auf diese Weise kann die funktionelle Ankopplung der externen Funktionseinrichtung zwischen dem ersten Kontaktabschnitt und dem zweiten Kontaktabschnitt in vorteilhafter Weise weiter sichergestellt werden.

Die Verriegelungseinrichtung kann mit der Trägereinrichtung verbunden sein. Die Verpresseinrichtung kann den zweiten Kontaktabschnitt über die Verriegelungseinrichtung gegen den ersten Kontaktabschnitt verpressen.

In einer weiter bevorzugten Ausführungsform weist die Verriegelungseinrichtung wenigstens eine erste Erfassungseinrichtung zum Erfassen einer Position und/oder eines Zustands des ersten Kontaktabschnitts und/oder des zweiten Kontaktabschnitts auf.

Eine "Erfassungseinrichtung", wie sie hierin eingesetzt werden kann, kann beispielsweise ein Sensor und/oder Detektor sein, der eine Position und/oder einen Zustand des ersten Kontaktabschnitts und/oder des zweiten Kontaktabschnitts erkennt und gegebenenfalls auswertet.

Eine "Position und/oder ein Zustand" des ersten Kontaktabschnitts und/oder des zweiten Kontaktabschnitts kann eine räumliche Anordnung und/oder Ausrichtung des ersten Kontaktabschnitts und/oder des zweiten Kontaktabschnitts bezeichnen. Sie kann eine Anordnung des ersten Kontaktabschnitts und des zweiten Kontaktabschnitts relativ zueinander bezeichnen.

Die Erfassungseinrichtung und/oder die Vorrichtung und/oder die Anordnung kann Einrichtungen zum Auswerten und/oder Steuern und/oder Regeln, insbesondere zum Korrigieren, der Position und/oder des Zustands des ersten Kontaktabschnitts und/oder des zweiten Kontaktabschnitts aufweisen. Derartige Einrichtungen können untereinander in Signalkommunikation stehen.

Die Verriegelungseinrichtung kann eine geeignete Sensoranordnung zum Erkennen der Zustände des zweiten Kontaktabschnitts, wie beispielsweise eines Zustands "Tür geschlossen" und/oder "Tür verriegelt", aufweisen. Erfindungsgemäß können eine Anzeige- und/oder eine Alarmeinrichtung zum Kommunizieren des erkannten Zustands vorgesehen sein.

In einer weiteren bevorzugten Ausführungsform sind der erste Kontaktabschnitt und/oder der zweite Kontaktabschnitt über ein Gelenk mit der Trägereinrichtung, der Vorrichtung oder der Anordnung verbunden.

Ein "Gelenk", wie es erfindungsgemäß verwendet kann, kann ein stabiles Gelenk, wie beispielsweise ein Scharnier, ein Türgelenk, einen Hebel und dergleichen bezeichnen.

Ein Türgelenk ist vorzugsweise einachsig ausgeführt.

Der über ein derartiges Gelenk mit der Trägereinrichtung verbundene Kontaktabschnitt kann vorzugsweise um eine sich beispielsweise in vertikaler Richtung erstreckenden Achse drehbar sein. Er kann insbesondere in Form einer Tür und/oder Abdeckung auf- und zuklappbar sein.

In einer wiederum weiter bevorzugten Ausführungsform ist wenigstens einer der Kontaktabschnitte oder eine Kontaktfläche oder eine Anpressfläche hiervon drehbar oder schwenkbar mit der Vorrichtung oder der Anordnung verbunden - beispielsweise mittels einer Tür oder als Teil einer Tür. Dabei liegt die Drehachse - beispielsweise als Drehachse eines Türgelenks der Tür - in einer Ebene mit - und vorzugsweise zudem parallel zu - einer Drehachse eines in dieser Ausführungsform drehbar gelagerten anderen Kontaktabschnitts.

In einer weiter bevorzugten Ausführungsform weisen der erste Kontaktabschnitt und/oder der zweite Kontaktabschnitt und/oder das Gelenk wenigstens eine Einrichtung zum Aufnehmen von Anbindungselementen zum funktionellen Anbinden der externen Funktionseinrichtung an die Anordnung auf.

Eine solche "Einrichtung zum Aufnehmen von Anbindungselementen" kann eine Öffnung, insbesondere eine sich durch die Komponente erstreckende Öffnung, wie beispielsweise eine Durchführung sein. Sie kann in ihrem Inneren zum Aufnehmen von Leitungen, Führungen, Zügen oder dergleichen ausgestaltet sein.

"Anbindungselemente", wie sie im Rahmen der vorliegenden Erfindung in Betracht gezogen werden, können Leitungen, wie elektrische und/oder pneumatische Leitungen, Kabel, wie Glasfaserkabel, Fluidleitungen und dergleichen, umfassen.

Derartige Anbindungselemente können die externe Funktionseinrichtung beispielsweise mit Druck versorgen. Ferner können über sie auch Sensoren und/oder an die externe Funktionseinrichtung angekoppelt werden.

In einer weiter bevorzugten Ausführungsform weisen der erste Kontaktabschnitt und/oder der zweite Kontaktabschnitt wenigstens einen transparenten Abschnitt auf.

Der Begriff "transparent", wie er in der vorliegenden Erfindung verwendet wird, bezeichnet eine - insbesondere für das menschliche Auge - durchsichtig ausgelegte Komponente.

Ein "transparenter Abschnitt" kann, ohne darauf beschränkt zu sein, aus Glas, durchsichtigem Kunststoff, durchsichtiger Folie oder dergleichen ausgebildet sein oder dergleichen umfassen.

Ein transparenter Abschnitt kann in vorteilhafter weise ein Betrachten und/oder optisches Überwachen eines Inneren der Anordnung zulassen. Er kann opak sein.

Ein Kontaktabschnitt, zum Beispiel der zweite Kontaktabschnitt, insbesondere wenn in Form einer Tür ausgestaltet, kann eine stabile Trägerstruktur mit einer in Teilbereichen vorgesehenen transparenten Abdeckung aufweisen. Eine derartige Ausführung kann einem Anwender der Anordnung in vorteilhafter weise erlauben, interessierende Bereiche der externen Funktionseinrichtung optisch von außen zu überwachen.

In einer weiter bevorzugten Ausführungsform können der erste Kontaktabschnitt und/oder der zweite Kontaktabschnitt wenigstens eine Positionierungseinrichtung zum Positionieren der externen Funktionseinrichtung innerhalb der Vorrichtung aufweisen.

Eine "Positionierungseinrichtung" im Sinne der vorliegenden Erfindung kann als eine zum definierten Aufnehmen und/oder räumlichen Positionieren und/oder kraft- und/oder formschlüssigen Befestigen der externen Funktionseinrichtung am Kontaktabschnitt ausgestaltet sein. Sie kann beispielsweise als Positionierstift, Positionierdorn und dergleichen ausgestaltet sein.

Die externe Funktionseinrichtung kann wenigstens eine Einrichtung zum Aufnehmen der Positioniereinrichtung, wie beispielsweise eine Öffnung, insbesondere eine Durchgangsöffnung, aufweisen.

Das Positionieren der externen Funktionseinrichtung innerhalb der Vorrichtung kann beispielsweise nach dem so genannten Loch-Langloch-Prinzip mittels geeigneter Einrichtungen erfolgen.

In einer weiter bevorzugten Ausführungsform weist die Vorrichtung wenigstens eine Abdichtungseinrichtung auf.

Eine solche "Abdichtungseinrichtung" kann zum Abdichten eines ersten Volumens der Vorrichtung gegen ein zweites Volumen der Vorrichtung geeignet sein.

Eine solche Abdichtungseinrichtung kann eine Matte sein. Sie kann eine Gummimatte sein. Sie kann als Mehrkomponenten-Abdichtungseinrichtung ausgelegt sein. Sie kann wenigstens ein elastomeres Material aufweisen. Sie kann zwischen dem ersten Kontaktabschnitt und dem zweiten Kontaktabschnitt angeordnet sein. Sie kann für den Gebrauchszustand der externen Funktionseinrichtung bzw. für den Verbindungszustand zwischen einem bewegbaren Kontaktabschnitt und der externen Funktionseinrichtung vorgesehen sein.

Die Abdichtungseinrichtung kann Sensoren und/oder Aktoren und/oder Leitungen und/oder Teile derselben aufweisen und/oder zum Aufnehmen derselben ausgelegt sein. Die Abdichtungseinrichtung kann mit derartigen Komponenten integriert ausgebildet sein. Die Komponenten können form- und/oder kraft- und/oder stoffschlüssig mit der Abdichtungseinrichtung verbunden sein.

Zu den geeigneten Abdichtungseinrichtungen zählen solche, wie sie in einer der Druckschriften DE 10 2007 042 964, DE 10 2008 062 037 oder der am selben Anmeldetag wie die vorliegende Anmeldung von der vorliegenden Anmelderin beim Deutschen Patent- und Markenamt angemeldeten Patentanmeldung DE 10 2009 012 632.5 mit dem Titel "Abdichtungseinrichtung zum Abdichten eines Volumens einer medizinischen Behandlungsanordnung gegen ein weiteres Volumen sowie Anordnung und Verfahren" offenbart sind. Auf die diesbezügliche Offenbarung der genannten Patentanmeldungen wird hierin vollinhaltlich Bezug genommen.

Die erfindungsgemäße Aufgabe wird ferner durch ein Verfahren gemäß dem Anspruch 13 gelöst.

Ein solches Verfahren umfasst das Verbinden wenigstens einer externen Funktionseinrichtung mit der Anordnung unter Verwenden der erfindungsgemäßen Vorrichtung.

Die erfindungsgemäße Aufgabe wird ebenso durch eine Anordnung gemäß dem Anspruch 14 gelöst. Eine solche erfindungsgemäße Anordnung weist wenigstens eine erfindungsgemäße Vorrichtung auf. In einer bevorzugten Ausführungsform ist die erfindungsgemäße Anordnung eine Blutbehandlungsvorrichtung, insbesondere eine Dialysevorrichtung, eine Hämodialysevorrichtung, eine Hämodiafiltrationsvorrichtung oder dergleichen.

Zur Vermeidung von Wiederholungen wird an dieser Stelle auf die vorstehend beschriebenen Ausführungen verwiesen, die ungeschmälert auf jegliche Ausführungsformen des erfindungsgemäßen Verfahrens und der erfindungsgemäßen Anordnung zutreffen. Zudem sind die genannten Vorteile ungeschmälert erzielbar.

Die erfindungsgemäße Vorrichtung ist vorteilhaft zum präzisen Ankoppeln einer externen Funktionseinrichtung an eine Anordnung geeignet. Durch den wenigstens einen, beispielsweise in einer Rotationsrichtung, beweglich ausgestalteten Kontaktabschnitt kann das Ausgleichen auftretender Toleranzen zwischen den einzelnen Bauteilen möglich sein und eine optimale mechanische und/oder funktionelle Ankopplung sichergestellt werden. Aufwändige Ankoppelvorgänge, wie sie beispielsweise in der Patentanmeldung mit der Veröffentlichungsnummer DE 10 2007 04 29 64 beschrieben sind, deren diesbezügliche Offenbarung durch Verweis hierin wiederum aufgenommen ist, ist mittels der erfindungsgemäßen Vorrichtung vorteilhaft auf einfache, schnelle und fehlerfreie Weise möglich.

Die durch Fertigungstoleranzen, elastisches Werkstoffverhalten sowie Verschleiß der einzelnen Komponenten im Laufe ihrer Lebensdauer allgemein erschwerte, sichere und dichte Ankopplung - beispielsweise unter der Forderung gleichmäßig über die Kontaktflächen verteilten hohen Drucks - kann durch die Beweglichkeit und Nachgiebigkeit bzw. Selbstnachführung eines Kontaktabschnitts in wenigstens einer Raumrichtung in vorteilhafter Weise sichergestellt werden.

Auf Grund der Möglichkeit der selbsttätigen Anpassung eines Kontaktabschnitts kann es in vorteilhafter Weise möglich sein, eine geeignete, sichere und fehlerfreie sowie verschleißarme Ankopplung der Sensoren und Aktoren der Ankoppelfläche - auch als Maschineninterface bezeichnet - und damit die angestrebte Funktion derselben mit Bezug auf ihre Koppelpartner der externen Funktionseinrichtung - und umgekehrt - sicherzustellen.

Da alle wesentlichen Komponenten auf einer einzigen, stabilen Trägereinrichtung montiert und positioniert sein können, kann in vorteilhafter Weise eine präzise Verpressung der Bauteile mit relativ hohen Kräften erreicht werden.

Aufgrund der Beweglichkeit oder Drehbarkeit des Kontaktabschnitts um eine Achse kann eine Verpresskraft auf vorteilhafte Weise gleichmäßig, auf eine Fläche oder auf mehrere Punkte verteilt werden. Daher können bei Bedarf vorteilhaft auch vergleichsweise hohe Kräfte aufgebracht werden. Die Beweglichkeit oder Drehbarkeit um eine Achse kann sicherstellen, dass es zu keinen unzulässigen Spitzenbelastungen durch Druckaufbringung kommt.

Dies gilt insbesondere, wenn mittels der erfindungsgemäß vorgesehenen drehbaren Anordnung wenigstens eines Kontaktabschnitts, vorzugsweise zwei Kontaktabschnitten, um jeweils eine Achse in vorteilhafter Weise die Kontaktflächen stets zueinander parallel ausgerichtet sein können. Dies erlaubt wiederum eine optimale Ankopplung der wenigstens einen externen Funktionseinrichtung bei Vermeidung von Belastungsspitzen. Zudem kann eine besonders präzise Mechanik, mittels welcher die Kontaktabschnitte zum verbinden der externen Funktionseinrichtung mit der Vorrichtung oder der Anordnung aufeinander zu geführt werden, ermöglicht werden.

Durch geeignete Anordnung von Lagerungspunkten des ersten und zweiten Kontaktabschnitts kann daneben in vorteilhafter Weise eine mögliche, durch die Verpresskraft zum Ankoppeln der externen Funktionseinrichtung hervorgerufene Durchbiegung der Komponenten kompensiert werden.

Die mögliche Beweglichkeit oder Pendelbewegung eines Kontaktabschnitts kann Bautoleranzen der einzelnen Komponenten, des ersten und/oder zweiten Kontaktabschnitts, der Abdichtungseinrichtung ausgleichen und in vorteilhafter Weise eine Abnutzung derselben verringern oder im Wesentlichen vermeiden.

Im Folgenden wird die erfindungsgemäße Vorrichtung anhand einer bevorzugten Ausführungsform derselben unter Bezugnahme auf die Figuren 1 bis 3 beschrieben. In der Zeichnung bezeichnen gleiche Bezugszeichen gleiche oder identische Elemente. Es gilt:
- Fig. 1: zeigt eine schematische Seitenansicht der erfindungsgemäßen Vorrichtung gemäß der bevorzugten Ausführungsform im Schnitt;
- Fig. 2: zeigt eine schematische Vorderansicht der erfindungsgemäßen Vorrichtung der Figur 1; und
- Fig. 3: zeigt eine weitere schematische Vorderansicht der erfindungsmäßen Vorrichtung der Figuren 1 und 2.

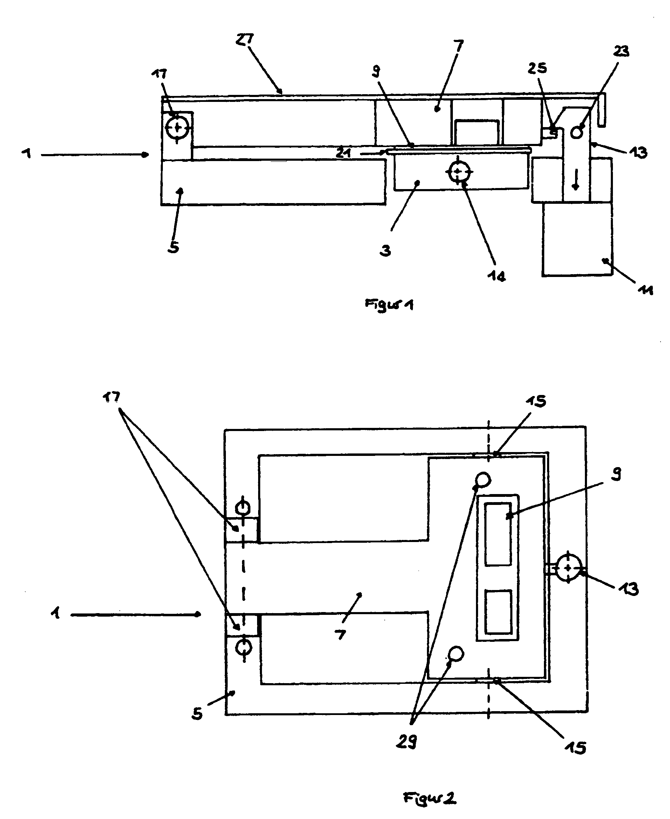

Fig. 1 zeigt schematisch eine Seitenansicht einer erfindungsgemäßen Vorrichtung 1 in einer Schnittdarstellung. Die Vorrichtung 1 weist einen ersten Kontaktabschnitt 3 in Form einer AS-Platte auf, die vertikal pendelnd oder drehbar in einer Trägereinrichtung 5, hier ausgestaltet als ein stabiler, umlaufender Trägerrahmen, gelagert ist.

An der Trägereinrichtung 5 ist über ein Gelenk 17 ein zweiter Kontaktabschnitt 7, hier eine stabile Tür, befestigt. Der zweite Kontaktabschnitt 7 dient zum Abdecken und zum Einspannen einer externen Funktionseinrichtung 9, hier in Gestalt einer blutführenden Disposable-Kassette.

Zur zusätzlichen Unterstützung der Einspannung und/oder der Verpressung der externen Funktionseinrichtung 9 ist eine an der Trägereinrichtung 5 befestigte Verpressungseinrichtung 11, hier ein Verpressaktor, angeordnet. Die Verpressungseinrichtung 11 kann dazu ausgelegt sein, die Tür als zweiten Kontaktabschnitt 7 über eine Verriegelungseinrichtung 13, hier einen Riegel, gegen die AS-Platte als ersten Kontaktabschnitt 3 zu verpressen. Ein Magnet 25 zum Halten der Tür, vor allem in einem Zustand ohne eingelegte externe Funktionseinrichtung 9, ist zwischen dem Riegel als Verriegelungseinrichtung 13 und der Disposable-Kassette als externen Funktionseinrichtung 9 angeordnet.

Der erste Kontaktabschnitt 3 ist mittels einer Achse oder Welle 14 an zwei Lagerungspunkten 15 an seiner schmalen Seite (seine obere und seine untere Seite in Fig. 2) in der Trägereinrichtung 5 gelagert.

Das Gelenk 17 und die Verriegelungseinrichtung 13 des zweiten Kontaktabschnitts 7 sind jeweils mittig an der Trägereinrichtung 5 angebracht und verbinden den zweiten Kontaktabschnitt 7 mit der Trägereinrichtung 5.

Die Verriegelungseinrichtung 13 weist einen integrierten Sensor als Erfassungseinrichtung 23 auf. Der Sensor kann zum Beispiel erkennen, ob die Tür als zweiter Kontaktabschnitt 7 geschlossen ist.

Über bzw. vor der Tür ist eine Tür-Abdeckung 27 angebracht. Die Tür-Abdeckung 27 kann transparente Bereiche aufweisen.

Die Fig. 2 zeigt eine Vorderansicht der erfindungsgemäßen Vorrichtung der Fig. 1 ohne Tür-Abdeckung 27. Die Tür weist Anbindungselemente 29 zum Versorgen der Disposable-Kassette mit Druck auf.

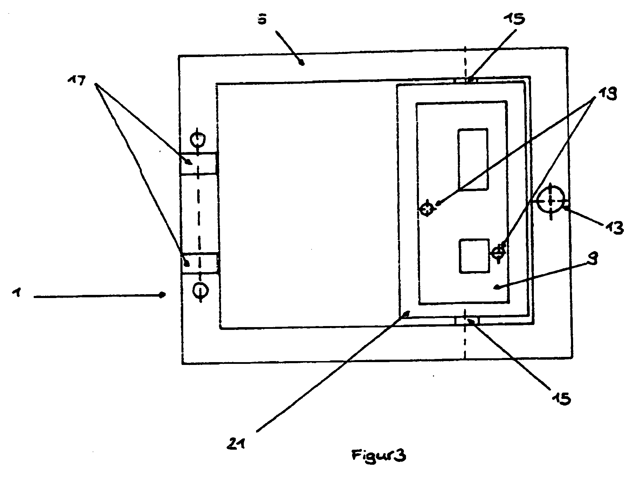

Die Fig. 3 zeigt die erfindungsgemäße Vorrichtung der Fig. 1 ohne die Tür und ohne die Tür-Abdeckung 27.

In Fig. 3 ist ferner eine Matte als Abdichteinrichtung 21 unterhalb der Disposable-Kassette auf der AS-Platte (letztere ist in Fig. 3 nicht zu sehen, da von der Disposable-Kassette und der Matte verdeckt) angeordnet.

In Fig. 3 sind Öffnungen 19 zum Aufnehmen von Befestigungseinrichtungen, wie beispielsweise Positionierdornen (nicht gezeigt), dargestellt. Solche Positionierdorne können zum Fixieren der externen Funktionseinrichtung am ersten Kontaktabschnitt nach dem Loch-Langloch-Prinzip geeignet zu sein.

Die vorliegende Erfindung ist nicht auf die vorstehend beschriebene Ausführungsform beschränkt, diese dient lediglich der Veranschaulichung.

## Patentansprüche

1. Vorrichtung (1) zum Verbinden wenigstens einer externen Funktionseinrichtung (9) mit einer Anordnung, wobei die Anordnung eine Blutbehandlungsvorrichtung ist und wobei die Vorrichtung (1) aufweist:
- wenigstens eine Aufnahmeeinrichtung mit wenigstens einem ersten Kontaktabschnitt (3) und wenigstens einem zweiten Kontaktabschnitt (7) zum Aufnehmen der externen Funktionseinrichtung (9) zwischen dem ersten Kontaktabschnitt (3) und dem zweiten Kontaktabschnitt (7) ;
wobei der erste Kontaktabschnitt (3) und der zweite Kontaktabschnitt (7) bewegbar angeordnet sind;
wobei der zweite Kontaktabschnitt (7) um wenigstens eine zweite Achse (17) drehbar gelagert ist;
**dadurch gekennzeichnet, dass**
der erste Kontaktabschnitt (3) um wenigstens eine erste zur zweiten Achse (17) parallele Achse (14) oder mittels wenigstens eines Kugelgelenks drehbar gelagert ist.

2. Vorrichtung (1) nach Anspruch 1 mit wenigstens einer umlaufend ausgestalteten Trägereinrichtung (5), wobei der erste Kontaktabschnitt (3) in einem Inneren der Trägereinrichtung (5) gelagert ist.

3. Vorrichtung (1) nach einem der vorangegangenen Ansprüche, wobei der zweite Kontaktabschnitt (7) mittels wenigstens eines Kugelgelenks gelagert ist.

4. Vorrichtung (1) nach einem der vorangegangenen Ansprüche, wobei wenigstens eine Kontaktfläche des ersten und/oder des zweiten Kontaktabschnitts (3, 7) und wenigstens eine Kontaktfläche der externen Funktionseinrichtung (9) angeordnet sind, um in einem angekoppelten Zustand der externen Funktionseinrichtung (9) plan aufeinander zu liegen.

5. Vorrichtung (1) nach einem der vorangegangenen Ansprüche, mit wenigstens einer Verpresseinrichtung (11) zum Verpressen der externen Funktionseinrichtung (9) zwischen dem ersten Kontaktabschnitt (3) und dem zweiten Kontaktabschnitt (7).

6. Vorrichtung (1) nach Anspruch 5, welche ferner wenigstens eine Verriegelungseinrichtung (13) zum Fixieren des verpressten Zustands der externen Funktionseinrichtung (9) zwischen dem ersten Kontaktabschnitt (3) und dem zweiten Kontaktabschnitt (7) aufweist.

7. Vorrichtung (1) nach Anspruch 6 wobei die Verriegelungseinrichtung (13) wenigstens eine erste Erfassungseinrichtung (23) zum Erfassen einer Position und/oder eines Zustands des ersten Kontaktabschnitts (3) und/oder des zweiten Kontaktabschnitts (7) aufweist.

8. Vorrichtung (1) nach Anspruch 2, wobei der erste Kontaktabschnitt (3) und/oder der zweite Kontaktabschnitt (7) über ein Gelenk (14, 17) mit der Trägereinrichtung (5) verbunden ist.

9. Vorrichtung (1) nach Anspruch 8, wobei der erste Kontaktabschnitt (3) und/oder der zweite Kontaktabschnitt (7) und/oder das Gelenk (17) wenigstens eine Einrichtung zum Aufnehmen von Anbindungselementen (29) zum funktionellen Anbinden der externen Funktionseinrichtung (9) an eine Anordnung aufweist.

10. Vorrichtung (1) nach einem der vorangegangenen Ansprüche, wobei der erste Kontaktabschnitt (3) und/oder der zweite Kontaktabschnitt (7) wenigstens einen transparenten Abschnitt aufweist.

11. Vorrichtung (1) nach einem der vorangegangenen Ansprüche, wobei der erste Kontaktabschnitt (3) und/oder der zweite Kontaktabschnitt (7) wenigstens eine Positionierungseinrichtung zum Positionieren der externen Funktionseinrichtung (9) innerhalb der Vorrichtung (1) aufweist.

12. Vorrichtung (1) nach einem der vorangegangenen Ansprüche, ferner mit wenigstens einer Abdichtungseinrichtung (21), welche zwischen wenigstens dem ersten Kontaktabschnitt (3) und dem zweiten Kontaktabschnitt (7) angeordnet ist.

13. Verfahren zum Verbinden wenigstens einer externen Funktionseinrichtung (9) mit einer Anordnung (1), **gekennzeichnet durch** den Schritt:
- Verwenden einer Vorrichtung (1) gemäß einem der Ansprüche 1 bis 12.

14. Anordnung mit wenigstens einer Vorrichtung (1) gemäß einem der Ansprüche 1 bis 12, wobei die Anordnung eine Blutbehandlungsvorrichtung ist.

## Claims

1. A device (1) for connecting at least one external functional means (9) to an arrangement, wherein the arrangement is a blood treatment apparatus and wherein the device (1) includes:
- at least one reception means having at least one first contact portion (3) and at least one second contact portion (7) for receiving the external functional means (9) between the first contact portion (3) and the second contact portion (7);
wherein the first contact portion (3) and the second contact portion (7) are disposed so as to be movable;
wherein the second contact portion (7) is mounted so as to be rotatable about at least one second axis (17);
**characterized in that**
the first contact portion (3) is mounted so as to be rotatable at least about a first axis (14) being parallel to the second axis (17) or with the aid of at least one ball-and-socket joint.

2. The device (1) according to claim 1, having at least one peripheral supporting means (5), wherein the first contact portion (3) is mounted in an interior of the supporting means (5).

3. The device (1) according to any one of the preceding claims, wherein the second contact portion (7) is mounted with the aid of at least one ball-and-socket joint.

4. The device (1) according to any one of the preceding claims, wherein at least one contact surface of the first and/or of the second contact portions (3, 7) and at least one contact surface of the external functional means (9) are disposed such that they come to lie on each other in a planar manner in a coupled condition of the external functional means (9).

5. The device (1) according to any one of the preceding claims, having at least one pressing means (11) for pressing the external functional means (9) between the first contact portion (3) and the second contact portion (7).

6. The device (1) according to claim 5, which further includes at least one locking means (13) for fixation of the pressed condition of the external functional means (9) between the first contact portion (3) and the second contact portion (7).

7. The device (1) according to claim 6, wherein the locking means (13) includes at least one first detection means (23) for detecting a position and/or a condition of the first contact portion (3) and/or of the second contact portion (7).

8. The device (1) according to claim 2, wherein the first contact portion (3) and/or the second contact portion (7) is connected to the supporting means (5) by using an articulation (14, 17).

9. The device (1) according to claim 8, wherein the first contact portion (3) and/or the second contact portion (7) and/or the articulation (17) includes at least one means for receiving linking elements (29) for functionally linking the external functional means (9) to an arrangement.

10. The device (1) according to any one of the preceding claims, wherein the first contact portion (3) and/or the second contact portion (7) includes at least one transparent portion.

11. The device (1) according to any one of the preceding claims, wherein the first contact portion (3) and/or the second contact portion (7) includes at least one positioning means for positioning the external functional means (9) inside the device (1).

12. The device (1) according to any one of the preceding claims, further having at least one sealing means (21) which is disposed between at least the first contact portion (3) and the second contact portion (7).

13. A method for connecting at least one external functional means (9) to an arrangement, **characterized by** the step of:
- using a device (1) according to any one of claims 1 to 12.

14. An arrangement having at least one device (1) according to any one of claims 1 to 12.

## Revendications

1. Un dispositif (1) destiné à connecter au moins un moyen fonctionnel externe (9) avec un arrangement, l'arrangement étant un appareil de traitement du sang et le dispositif (1) comprenant:
- au moins un moyen de réception ayant au moins une première section de contact (3) et au moins une seconde section de contact (7) destinées à recevoir le moyen fonctionnel externe (9) entre la première section de contact (3) et la seconde section de contact (7);
où la première section de contact (3) et la seconde section de contact (7) sont agencées de sorte à être mobiles;
où la seconde section de contact (7) est montée de sorte à pouvoir pivoter au moins autour d'un second axe (17);
**caractérisé en ce que**
la première section de contact (3) est montée de sorte à pouvoir pivoter au moins autour d'un premier axe (14) parallèle au second axe (17) ou au moyen d'une rotule.

2. Le dispositif (1) selon la première revendication, ayant au moins un moyen de support (5) périphérique, où la première section de contact (3) est montée dans un intérieur du moyen de support (5).

3. Le dispositif (1) selon l'une des revendications précédentes, où la seconde section de contact (7) est montée au moyen d'au moins une rotule.

4. Le dispositif (1) selon l'une des revendications précédentes, où au moins une surface de contact de la première et/ou de la seconde section de contact (3, 7) et au moins une surface de contact du moyen fonctionnel externe (9) sont agencées de sorte à reposer l'une sur l'autre de façon planaire dans un état couplé du moyen fonctionnel externe (9).

5. Le dispositif (1) selon l'une des revendications précédentes ayant au moins un moyen de pressage (11) destiné à presser le moyen fonctionnel externe (9) entre la première section de contact (3) et la seconde section de contact (7).

6. Le dispositif (1) selon la revendication 5 comprenant de plus au moins un moyen de verrouillage (13) destiné à fixer l'état pressé du moyen fonctionnel externe (9) entre la première section de contact (3) et la seconde section de contact (7).

7. Le dispositif (1) selon la revendication 6, où le moyen de verrouillage (13) comprend au moins un premier moyen de saisie (23) destiné à saisir une position et/ou un état de la première section de contact (3) et/ou de la seconde section de contact (7).

8. Le dispositif (1) selon la revendication 2, où la première section de contact (3) et/ou la seconde section de contact (7) est connectée avec le moyen de support (5) au moyen d'une articulation (14, 17).

9. Le dispositif (1) selon la revendication 8, où la première section de contact (3) et/ou la seconde section de contact (7) et/ou l'articulation (17) comprend au moins un moyen destiné à recevoir des éléments de raccord (29) destinés à raccorder fonctionnellement le moyen fonctionnel externe (9) avec un arrangement.

10. Le dispositif (1) selon l'une des revendications précédentes, où la première section de contact (3) et/ou la seconde section de contact (7) comprend au moins une section transparente.

11. Le dispositif (1) selon l'une des revendications précédentes, où la première section de contact (3) et/ou la seconde section de contact (7) comprend au moins un moyen de positionnement destiné à positionner le moyen fonctionnel externe (9) à l'intérieur du dispositif (1).

12. Le dispositif (1) selon l'une des revendications précédentes comprenant de plus au moins un moyen d'étanchéité (21) lequel est agencé entre au moins la première section de contact (3) et la seconde section de contact (7).

13. Un procédé destiné à connecter au moins un moyen fonctionnel externe (9) avec un arrangement **caractérisé par** l'étape :
- utiliser un dispositif (1) selon l'une des revendications 1 à 12.

14. Un arrangement ayant au moins un dispositif (1) selon l'une des revendications 1 à 12, où l'arrangement est un appareil de traitement du sang.
